# EUROPEAN PATENT APPLICATION

(11) **EP 3 195 802 A1**
(43) Date of publication of application: **26.07.2017**
(21) Application number: 16151878.2
(22) Date of filing: 19.01.2016
(51) Int. Cl.: A61B 6/03, A61B 6/12, A61B 6/00, G06T 7/00, G06T 7/60, G01N 23/04, G01B 21/04

(54) **GEOMETRICAL CALIBRATION OF X-RAY CT SCANNERS**

(71) Applicant: Danmarks Tekniske Universitet, 2800 Kgs. Lyngby (DK)
(72) Inventor: Stolfi, Alessandro, 2800 Kgs. Lyngby (DK); De Chiffre, Leonardo, 2700 Brønshøj (DK)
(74) Representative: Plougmann Vingtoft a/s

(57) **Abstract**

A method of performing calibration scan and measurement scan in one and the same scanning operation with a calibration object having the fiducial marks arranged in positions spanning a volume enclosing at least a central portion of the measuring object. This avoids the need for one or more separate calibration scans to be performed in addition to the scanning of the measurement object. Considerable time is thereby saved. The fiducial objects are thus distributed, preferably evenly, around the measuring object, whereby homogeneous calibration is ensured. After having performed a scan of the measuring object together with the calibration object and thereby obtained scan data on the measuring object and corresponding scan data on the calibration object the scan data on the fiducial marks of the calibration object are used to calibrate the CT scanner, and the scan data on the measuring object are used to calculate geometric properties of the measuring object.

## Description

### FIELD OF THE INVENTION

The invention relates to geometrical calibration of X-ray CT scanners in general and in particular to geometrical calibration of industrial X-ray CT scanners used in the field of dimensional metrology. Typical areas of use of CT in industry are in the detection of flaws such as voids and cracks and particle analysis in materials. In metrology CT allows measuring the external as well as the internal geometry of complex products. Thus, CT metrology can be used for nondestructive measurement of external and internal geometries.

### BACKGROUND OF THE INVENTION

Industrial CT scanners used in metrology provide high resolution in the order of micrometres. The procedure of measuring a measuring object in three dimensions is time consuming. Furthermore, to ensure accurate and reliable measuring results, the scanners need to be calibrated using a calibration object with precisely known geometry, typically involving physical elements such as spheres, which is also time consuming. In critical and demanding cases calibration is performed both before and after scanning the measuring object. The entire procedure including pre-calibration, scanning the measuring object and post calibration may last several hours.

It is therefore desirable to reduce the time for CT scanning a measuring object including the time for calibrating the CT scanner.

### SUMMARY OF THE INVENTION

The invention provides a method of performing calibration scan and measurement scan in one and the same scanning operation with a calibration object having the physical elements defining fiducial marks arranged in positions spanning a volume enclosing at least a central portion of the measuring object. This avoids the need for one or more separate calibration scans to be performed in addition to the scanning of the measurement object. Obviously, considerable time is thereby saved.

The fiducial marks are thus distributed around the measuring object, whereby homogeneous calibration is ensured. It is preferred that the volume spanned by the fiducial marks encloses the entire measuring object and not only a central portion.

In a preferred embodiment the calibration object comprises an X-ray transmissive tubular or ring-shaped supporting structure with the fiducial marks arranged in fixed positions on a surface of the supporting structure. Advantageously, the tubular or ring-shaped supporting structure includes a rigid base-element fixed to an end of the supporting structure.

In another preferred embodiment the calibration object comprises a rigid base with a plurality of X-ray transmissive elongate supporting elements extending from the base with the fiducial marks fixed to the supporting elements. This allows portions of the measuring object to extend through spaces between the supporting elements and outside the volume spanned by the fiducial marks.

An X-ray transmissive support can used to support the measuring object to ensure that the measuring object, or at least a central portion of it, is within the volume spanned by the fiducial marks.

After having performed a scan of the measuring object together with the calibration object and thereby obtained scan data on the measuring object and corresponding scan data on the calibration object the scan data on the fiducial marks of the calibration object are used to calibrate the CT scanner, and the scan data on the measuring object are used to calculate geometric properties of the measuring object.

### BRIEF DESCRIPTION OF THE DRAWINGS

Figure 1 shows a side view of a tubular calibration object according to the invention;
Figure 2 shows a view in the axial direction of the tubular calibration object in figure 1;
Figure 3 shows a side view of another calibration object according to the invention; and
Figure 4 shows a view in the axial direction of the calibration object in figure 4;

### DETAILED DESCRIPTION OF THE INVENTION

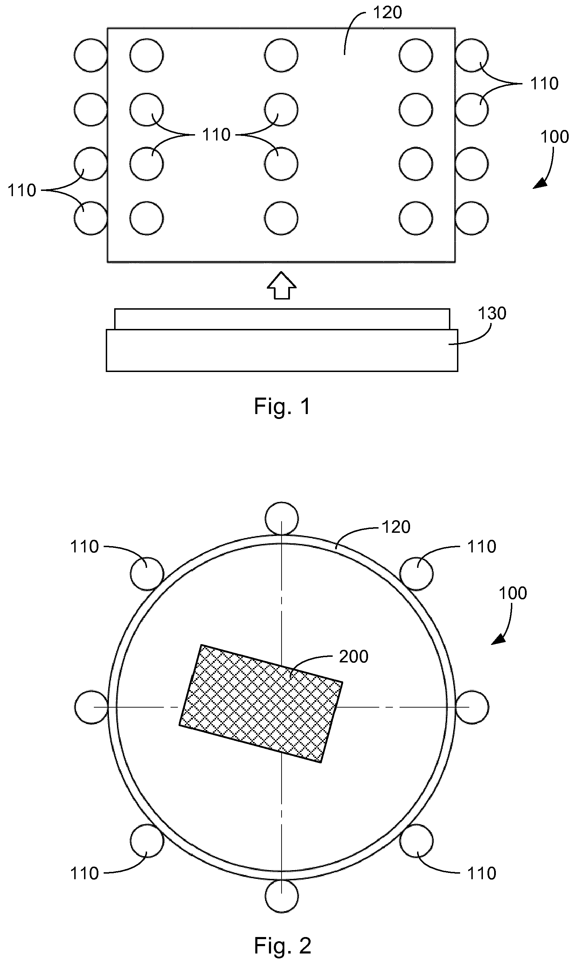

In figures 1 and 2 is shown a calibration object 100 with a plurality of possible positions for fiducial marks 110 secured to the outer surface of a tubular supporting structure 120 in precisely known positions relative to each other. Preferably, fiducial marks 110 should not be in positions on the calibration object where, at any time during scanning, a line through two or more of the fiducial marks will intersect the focal point of the X-ray source. This reduces the risk of image artefacts. The tubular supporting structure 120 is made of a rigid material with low X-ray absorption, such as carbon-fiber reinforced polymer, whereby image artefacts caused by X-ray absorption in the tubular supporting structure 120 are minimised. The fiducial marks 110 are preferably spherical elements with identical diameters and an X-ray transmissivity which matches that of the measuring object. Thus, when the measuring object is of a material with a relatively low X-ray transmissivity such as a polymer material, ruby is a preferred material for the fiducial marks 110. The tubular supporting structure 120 has a base 130 preferably with a stepped diameter fixed to one end of the tubular supporting structure 120 to further increase the mechanical stability of the calibration object 100. Preferred materials for the base include INVAR, which is a nickel-iron alloy notable for its uniquely low coefficient of thermal expansion, and carbon fibre reinforced polymer. The calibration object 100 as a whole as well as each of its components has high mechanical and thermal stability. In the shown example the tubular supporting structure 120 is in the shape of a circular cylinder, but other configurations are possible such as a polyhedron or a portion of a sphere. Figure 2 shows a measuring object 200 placed within the calibration object 100.

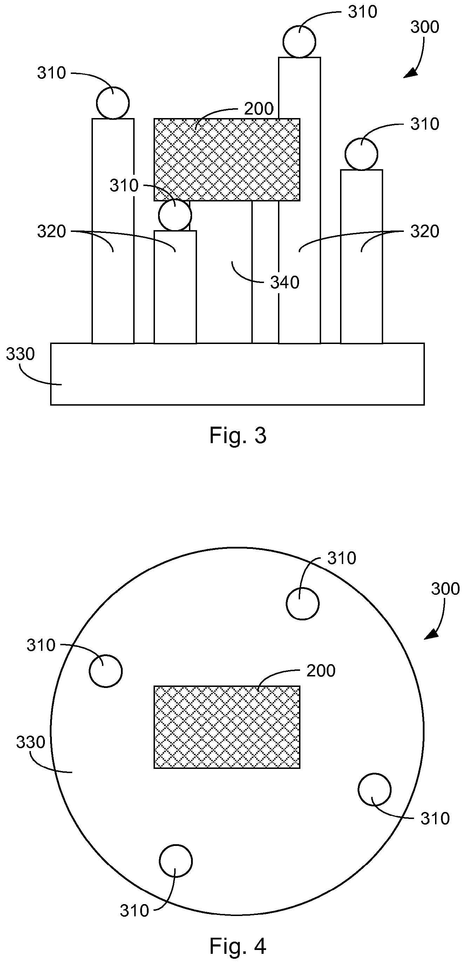

In figures 3 and 4 is shown another calibration object with four fiducial marks 310 arranged in precisely known positions relative to each other so that the fiducial marks define three linearly independent vectors, i.e. they are not included in the same plane. The calibration object 300 comprises a rigid base 330, preferably made of INVAR, with a plurality of X-ray transmissive elongate supporting elements 320 extending from the base with the fiducial marks 310 fixed to the supporting elements. A preferred material for the supporting elements 320 is carbon-fiber-reinforced polymer. Each of the supporting elements 320 can have one or more fiducial marks 310 fixed thereto, e.g. at an end as shown or at the side. A measuring object 200 placed within a volume spanned by the fiducial marks 310 of the calibration object 300. Advantageously, an X-ray transmissive support 340 is used to support the measuring object.

## Claims

1. A method of measuring three dimensional dimensions of a measuring object (200) using an X-ray CT scanner having a measuring volume, the method comprising
arranging the measuring object (200) together with a calibration object (100, 300) in the measuring volume;
scanning the measuring object (200) and the calibration object (100, 300) together in the measuring volume so as to obtain scan data on the measuring object (200) and corresponding scan data on the calibration object (100, 300); wherein
the calibration object (100, 300) has at least four fiducial marks (110, 310) arranged in known positions relative to each other, the fiducial marks (110, 310) defining at least three linearly independent vectors, and
the fiducial marks (110, 310) are arranged in positions spanning a volume enclosing at least a central portion of the measuring object (200).

2. A method according to claim 1 wherein the calibration object (100) comprises an X-ray transmissive tubular or ring-shaped supporting structure (120) with the fiducial marks (110) arranged in fixed positions on a surface of the supporting structure (120).

3. A method according to claim 2 wherein the tubular or ring-shaped supporting structure (120) includes a rigid base-element (130) fixed to an end of the supporting structure (120).

4. A method according to claim 1 wherein the calibration object (300) comprises a rigid base (330) with a plurality of X-ray transmissive elongate supporting elements (320) extending from the base (330) with the fiducial marks (310) fixed to the supporting elements (320).

5. A method according to any one of the preceding claims wherein an X-ray transmissive support (340) is used to support the measuring object.

6. A method according to any one of the preceding claims wherein the scan data on the fiducial marks of the calibration object (100, 300) are used to calibrate the CT scanner.

7. A method according to any one of the preceding claims wherein the scan data on the measuring object (200) are used to calculate geometric properties of the measuring object (200).
